(19) Europäisches Patentamt

European Patent Office

Office européen des brevets

(11) **EP 1 242 098 B1**

(12) **FASCICULE DE BREVET EUROPEEN**

| | |
|---|---|
| (45) Date de publication et mention de la délivrance du brevet: **01.08.2007 Bulletin 2007/31** | (51) Int Cl.: **A61K 35/08** (2006.01)  **A61K 33/14** (2006.01)  **A61P 27/02** (2006.01) |
| (21) Numéro de dépôt: **00990830.2** | (86) Numéro de dépôt international: **PCT/FR2000/003709** |
| (22) Date de dépôt: **28.12.2000** | (87) Numéro de publication internationale: **WO 2001/049304 (12.07.2001 Gazette 2001/28)** |

(54) **SOLUTIONS IONIQUES AQUEUSES ET LEURS UTILISATIONS NOTAMMENT EN OPHTALMOLOGIE**

IONISCHE WÄSSRIGE LÖSUNGEN UND DEREN VERWENDUNGEN INSBESONDERE IN DER OPHTALMOLOGIE

AQUEOUS IONIC SOLUTIONS AND THEIR USES IN PARTICULAR IN OPHTHALMOLOGY

| | |
|---|---|
| (84) Etats contractants désignés: **AT BE CH CY DE DK ES FI FR GB GR IE IT LI LU MC NL PT SE TR** | • **HALLEY, Bénédicte, Marie, Dominique F-14000 Caen (FR)**  • **LEROY, Didier F-35730 Pleurtuit (FR)** |
| (30) Priorité: **31.12.1999 FR 9916814** | |
| (43) Date de publication de la demande: **25.09.2002 Bulletin 2002/39** | (74) Mandataire: **Koch, Gustave Cabinet Plasseraud 52 rue de la Victoire 75440 Paris Cedex 09 (FR)** |
| (73) Titulaire: **LABORATOIRES GOEMAR S.A. 35400 Saint-Malo (FR)** | |
| (72) Inventeurs: • **YVIN, Jean-Claude F-35400 Saint Malo (FR)** | (56) Documents cités: **FR-A- 2 735 980** |

**Description**

L'invention concerne les solutions ioniques aqueuses du genre de celles qui sont obtenues notamment à partir d'eau de mer et leurs utilisations notamment en ophtalmologie.

Certaines de ces solutions ioniques aqueuses sont nouvelles. L'invention les vise par conséquent en tant que produits industriels nouveaux.

Mises à part ces dernières, les solutions ioniques aqueuses obtenues à partir d'eau de mer sont connues.

On a déjà proposé des les appliquer dans la prévention, l'hygiène et le traitement des voies respiratoires, buccales, de la peau et des muqueuses gynécologiques.

Il s'agissait là, en effet, des seules applications qui semblaient pouvoir être envisagées par l'homme du métier.

Or, la Société demanderesse a eu le mérite,

- d'une part, de trouver à l'issue de recherches approfondies que les solutions en question déjà connues, sous réserve d'une adaptation de leur composition, de leur pH et de leur osmolalité, pouvaient être utilisées pour le traitement et l'hygiène de l'oeil et de ses annexes et,
- d'autre part, de mettre au point des solutions ioniques aqueuses du genre en question, dont la composition ionique quantitative, le pH et l'osmolalité sont nouvelles,

L'invention a donc pour objet l'utilisation non thérapeutique pour l'hygiène de l'oeil et de ses annexes des solutions ioniques aqueuses obtenues à partir de l'eau de mer et dont la composition ionique, le pH et l'osmolalité ont été adaptées en conséquence.

L'invention a également pour objet l'utilisation des susdites solutions ioniques aqueuses pour la préparation d'un médicament destiné au traitement de l'oeil et de ses annexes.

Du point de vue qualitatif, la composition ionique des solutions mises en oeuvre conformément à l'invention est celle de l'eau de mer.

A titre illustratif, on indique dans le tableau ci-après la composition de l'eau de mer telle qu'elle figure à la page F 163 de l'ouvrage « Handbook of Chemistry and Physics » 63ème édition, 1982-1983, CRC PRESS.

TABLEAU

| Elément | Quantité (p.p.m.) |
|---|---|
| Cl | 18.980 |
| Na | 10.561 |
| Mg | 1.272 |
| s | 884 |
| Ca | 400 |
| K | 380 |
| Br | 65 |
| C (inorganique) | 28 |
| Sr | 13 |
| ($SiO_2$) | 0.01 - 7.0 |
| B | 4.6 |
| Si | 0.02-4.0 |
| C (organique) | 1.2-3.0 |
| Al | 0.16-1,9 |
| F | 1.4 |
| N(en tant que nitrate) | 0.001-0.7 |
| N (en tant qu'azote organique) | 0.03-0.2 |
| Rb | 0.2 |
| Li | 0.1 |

(suite)

| Elément | Quantité (p.p.m.) |
|---|---|
| P(en tant que phosphate) | >0.001-0.10 |
| Ba | 0.05 |
| I | 0.05 |
| N(en tant que nitrite) | 0.0001-0.05 |
| N (en tant qu' ammoniaque) | >0.005-0.05 |
| As (en tant qu' arsenic) | 0.003-0 ,024 |
| Fe | 0.002-0,02 |
| P(en tant que phosphore organique) | 0.016 |
| Zn | 0.005-0.014 |
| Cu | 0.001-0.09 |
| Mn | 0.001-0.01 |
| Pb | 0.004-0.005 |
| Se | 0.004 |
| Sn | 0.003 |
| Cs | 0.002 (approximativement) |
| U | 0.00015-0.0016 |
| Mo | 0.0003-0.002 |
| Ga | 0.0005 |
| Ni | 0.0001.0.0005 |
| Th | <0.0005 |
| Ce | 0.0004 |
| V | 0.0003 |
| La | 0.0003 |
| Y | 0.0003 |
| Hg | 0.00003 |
| Ag | 0.00015-0.0003 |
| Bi | 0.0002 |
| Co | 0.0001 |
| Sc | 0.00004 |
| Au | 0.000004-0.000008 |
| Fe (en solution vraie) | $<10^{-9}$ |
| Ra | $2.10^{-11}$-$3.10^{-10}$ |
| Ge | Présent |
| Ti | Présent |
| W | Présent |
| Cd | Présent dans des organismes marins |
| Cr | Présent dans des organismes marins |
| Tl | Présent dans des organismes marins |

(suite)

| Elément | Quantité (p.p.m.) |
|---------|-------------------|
| Sb | Présent dans des organismes marins |
| Zr | Présent dans des organismes marins |
| Pt | Présent dans des organismes marins |

A la même page du même ouvrage il est précisé que le pH de l'eau de mer est de 8-9.

On sait par ailleurs (IFREMER, Département Environnement Littoral et Gestion du milieu marin) que l'osmolalité de l'eau de mer est > 1000 mOsm/kg.

Du point de vue quantitatif, la composition ionique des solutions en question obtenues à partir de l'eau de mer est choisie de façon telle que leur pH soit de 4 à 9, de préférence de 7 à 8 et que leur osmolalité soit de 150 à 700, de préférence de 250 à 350 mOsm/kg.

Parmi les solutions ayant donné des résultats particulièrement encourageants dans l'utilisation conforme à l'invention on peut citer :

- les solutions ioniques aqueuses obtenues par dilution de l'eau de mer, en particulier avec de l'eau distillée, notamment à raison de 2 à 5 fois,
- les solutions ioniques aqueuses, obtenues à partir de l'eau de mer par les techniques connues sous le terme dessalage et, éventuellement, enrichies en au moins un de leurs ions,
- les solutions ioniques aqueuses obtenues artificiellement à partir de sels marins.

Les solutions ioniques aqueuses conformes à l'invention, qui constituent des produits industriels nouveaux et qui sont notamment obtenues à partir d'eau de mer, sont caractérisées par :

■ une valeur de pH de préférence inférieure ou au plus égale aux valeurs les plus basses du pH de l'eau de mer,
■ une osmolalité inférieure à celle de l'eau de mer et
■ une composition du point de vue ionique qui est qualitativement et quantitativement celle de l'eau de mer, à l'exception du point de vue quantitatif, d'une part, de la concentration en potassium qui est supérieure à celle de l'eau de mer et, d'autre part, des concentrations en Na, Mg, Ca et Cl qui sont inférieures à celles de l'eau de mer, lesdites concentrations étant

- pour $Na^+$, de 1300 à 1500, de préférence de 500 à 1000 mg/l,
- pour $K^+$, de 4500 à 6500, de préférence de 5000 à 6000 mg/l,
- pour $Mg^{++}$, de 50 à 1300, de préférence de 100 à 500 mg/l,
- pour $Ca^{++}$, de 20 à 350, de préférence de 40 à 200 mg/l,
- pour $Cl^-$, de 4000 à 6000, de préférence de 4500 à 5000 mg/l.

Dans le cas de l'eau de mer, les valeurs correspondantes sont matérialisées par les fourchettes reflétant les résultats de 134 mesures effectuées sur de l'eau de mer prélevée au large de Saint-Malo d'août 1998 à juillet 1999, à savoir :

pH : 7,70 à 8,30
osmolalité : > 1000 mOsm/kg
$[Na^+]$ : 10500-11500 mg/l
$[K^+]$ : 365-420 mg/l
$[Mg^{++}]$ : 1200-1450 mg/l
$[Ca^{++}]$ : 380-435 mg/l
$[Cl^-]$ : 18900-20500 mg/l

Les nouvelles solutions ioniques aqueuses conformes à l'invention sont particulièrement appropriées à l'hygiène de l'oeil et de ses annexes.

Par ailleurs, les nouvelles solutions ioniques aqueuses conformes à l'invention sont particulièrement appropriées pour la préparation d'un médicament destiné au traitement de l'oeil et de ses annexes.

Les solutions ioniques aqueuses conformes à l'invention telles que définies plus haut, lorsqu'elles sont mises en oeuvre dans l'utilisation non thérapeutique pour l'hygiène de l'oeil et de ses annexes, sont remarquables en ce

- qu'elles présentent le pH et l'osmolalité physiologique des larmes, et
- qu'elles sont exemptes d'agents conservateurs.

Ce dernier avantage est d'une grande importance.

En effet, les agents conservateurs présents dans la plupart des médicaments pour l'oeil sont considérés comme nocifs pour la cornée.

Pour la préparation de la solution ionique aqueuse conforme à l'invention, on a utilisé de l'eau de mer puisée au large de Saint-Malo et prélevée à une profondeur de 5 à 10 mètres dans une zone à forts mouvements de courant ; cette eau est caractérisée par une teneur en sels supérieure à 32 g/l ; elle est naturellement riche en calcium, magnésium et oligo-éléments.

Cette eau est soumise à une électrodialyse sélective ; dans un premier temps, on retire uniquement le chlorure de sodium pour atteindre l'osmolalité souhaitée, puis on ajuste les concentrations ioniques en fonction de l'utilisation thérapeutique ; le pH recherché est obtenu de préférence en échangeant les ions $Na^+$ contre des protons.

L'électrodialyse sélective peut être effectuée avec un appareil du type EUR 6B commercialisé par la Société EURODIA Industrie SA sous la marque EURODIA.

Les différentes étapes de l'électrodialyse sélective correspondant à l'ajustement à chacun des différents paramètres (pH, osmolalité, concentration en ions) sont effectuées de manière connue.

## EXEMPLE

Les caractéristiques de la solution examinée sont comme suit :

| | |
|---|---|
| pH : | 7,45 |
| osmolalité : | 309 mOsm/kg |
| $[Na^+]$ : | 680 mg/l |
| $[K^+]$ : | 5818 mg/l |
| $[Mg^{++}]$ : | 128 mg/l |
| $[Ca^{++}]$ : | 54 mg/l |
| $[Cl^-]$ : | 4850 mg/l. |

C'est à l'aide d'un test dérivé du test de Draize que l'on a montré que cette solution était peu irritante à l'égard de l'oeil et, en tout état de cause, moins irritante que le sérum physiologique.

Le test de Draize permet d'évaluer l'irritation oculaire après applications multiples ; il peut être mis en oeuvre sur l'oeil du lapin albinos.

La solution de référence utilisée dans ce test est constituée, comme indiqué ci-dessus, par le sérum physiologique, c'est-à-dire par une solution de chlorure de sodium (NaCl 0,9%) ; le sérum physiologique est traditionnellement utilisé pour rincer les yeux en cas de projection chimique accidentelle, pour nettoyer les yeux des nourrissons et également comme solvant des larmes artificielles, comme collyre ou comme solution de lavage ophtalmique ; c'est la solution de référence en ophtalmologie pour l'Agence Française de la Sécurité Sanitaire des Produits de Santé.

Selon le test de Draize, 12 lapins albinos sont répartis en deux groupes (un groupe de référence et un groupe pour la solution testée).

On administre, six fois de suite à 1 heure d'intervalle dans le cul-de-sac conjonctival de l'oeil droit

- d'une part, 50 μl de sérum physiologique dans le cas des lapins du groupe de référence et
- d'autre part, 50 μl de la solution testée dans le cas des lapins de l'autre groupe.

On examine les yeux traités des lapins à l'aide de la lampe à fente (Slit lamp AIT-20, Topcon, Topcon France - F-92300 Levallois-Perret), avant instillation, puis 1 heure après respectivement la 1ère et la 6ème instillation, puis 1, 2 et 3 jours après la sixième instillation.

On observe les effets produits sur la conjonctive, l'iris et la cornée.

En ce qui concerne la conjonctive, on relève :

(a) l'infiltration oedémateuse, en attribuant une note de 0 à 4, la note 0 désignant l'absence d'infiltration et la note 4 la fermeture complète des paupières,

(b) l'écoulement, en attribuant une note de 0 (aucun écoulement) à 3 (paupières et poils mouillés sur une large surface autour de l'oeil),

(c) la rougeur (c), en attribuant une note de 0 à 3, la note 0 désignant les vaisseaux normaux et la note 3 une rougeur intense de la conjonctive.

Pour l'évaluation de l'effet sur la conjonctive, on retient le chiffre obtenu par application de la formule :

$$(a + b + c) \times 2.$$

Dans le cas de l'iris , on attribue une note allant de 0 à 2, la note 0 correspondant à l'iris normal et la note 2 correspondant à un iris ne présentant aucune réaction à la lumière mais présentant, par contre, des hémorragies et altérations profondes.

Pour l'évaluation de l'effet sur l'iris, on retient le chiffre donné par la formule :

$$(d) \times 5.$$

En ce qui concerne enfin la cornée, on évalue, d'une part, l'intensité (e) de l'opacité, sachant que la note 0 est attribuée à l'absence d'opacité et la note 4 à une opacité cornéenne totale avec iris invisible et, d'autre part, la surface des opacités (f), les notes allant de 0 à 4, cette dernière note correspondant à une opacification de plus de ¾ de la surface totale.

Pour l'évaluation, on retient la valeur donnée par la formule :

$$e \times f \times 5.$$

La somme des valeurs obtenues pour la conjonctive, l'iris et la cornée, à chaque mesure et pour chaque animal, représente l'indice d'irritation individuel oculaire (IOI) qui est au maximum de 110.

La moyenne arithmétique des valeurs trouvées pour les 6 lapins représente l'indice d'irritation oculaire moyen (MOI).

L'indice d'irritation oculaire maximum (MOI max) est la valeur individuelle maximale obtenue à chaque mesure.

Les résultats obtenus avec la solution conforme à l'invention et avec le sérum physiologique sont réunis dans les tableaux I et II qui suivent et dans lesquels :

- Jour 1/0 désigne la mesure faite 1 jour avant l'instillation,
- Jour 1/1 désigne la mesure faite 1 heure après la 1ère instillation,
- Jour 1/6 désigne la mesure faite 1 heure après la 6ème instillation,
- Jour 2 désigne la mesure faite 24 heures après la dernière instillation,
- Jour 3 désigne la mesure faite 48 heures après la dernière instillation,
- Jour 4 désigne la mesure faite 72 heures après la dernière instillation.

**TABLEAU I.**

| (solution conforme à l'invention) | | | | | | |
|---|---|---|---|---|---|---|
| | **Jour 1/0** | **Jour 1/1** | **Jour 1/6** | **Jour 2** | **Jour 3** | **Jour 4** |
| **MOI** | 0,00 | 0,67 | 0,00 | 0,00 | 0,00 | 0,00 |
| **MOI max** | 0 | 4 | 0 | 0 | 0 | 0 |

**TABLEAU II**

| (sérum physiologique) | | | | | | |
|---|---|---|---|---|---|---|
| | **Jour 1/0** | **Jour 1/1** | **Jour 1/6** | **Jour 2** | **Jour 3** | **Jour 4** |
| **MOI** | 0,00 | 0,00 | 1,00 | 0,33 | 0,67 | 0,00 |

(suite)

| (sérum physiologique) | | | | | | |
|---|---|---|---|---|---|---|
| | Jour 1/0 | Jour 1/1 | Jour 1/6 | Jour 2 | Jour 3 | Jour 4 |
| MOI max | 0 | 0 | 4 | 2 | 2 | 0 |

En comparant ces valeurs, on constate que la supériorité de la solution conforme à l'invention se manifeste dès la mesure faite à Jour 1/6 et devient intéressante aux jours 2 et 3, ce qui signifie que la solution définie plus haut est particulièrement bénéfique pour les utilisations non thérapeutiques de longue durée et celles nécessitant des applications multiples.

Ceci étant, et d'un point de vue général, les produits, notamment ophtalmiques, préparés en ayant recours aux solutions ioniques aqueuses conformes à l'invention ou plus généralement à toutes les solutions ioniques aqueuses obtenues à partir d'eau de mer peuvent être présentés, par exemple, sous forme de lotions destinées au lavage de l'oeil, sous forme de collyres, de gels ophtalmologiques ou remplacer l'eau dans les inserts oculaires.

La composition d'une telle lotion peut être comme suit :

- Solution ionique aqueuse : q.s. 100%
- Acide salicylique: 0,1%
- Eau distillée d'hamamélis 0,4%

Les lotions en question peuvent être administrées en utilisant plus préférentiellement des dispositifs du genre de ceux selon la demande de brevet français déposée au nom de la Demanderesse le 13 octobre 1999 sous le No. 99 12782 sous le titre « Dispositif pour le lavage et le bain de l'oeil ».

A titre d'exemple, on signale que l'on peut procéder à 2 ou 3 lavages ou bains oculaires par jour.

**Revendications**

1. Utilisation non thérapeutique pour l'hygiène de l'oeil et de ses annexes des solutions ioniques aqueuses obtenues à partir de l'eau de mer, dont la composition ionique est du point de vue qualitatif celle de l'eau de mer et du point de vue quantitatif telle que d'une part leur pH soit de 4 à 9, de préférence de 7 à 8 et que d'autre part leur osmolalité soit de 150 à 700, de préférence de 250 à 350 mOsm/kg.

2. Utilisation selon la revendication 1 dans laquelle la solution ionique aqueuse est obtenue par dilution de l'eau de mer, en particulier avec de l'eau distillée, notamment à raison de 2 à 5 fois.

3. Utilisation selon la revendication 1 dans laquelle la solution ionique aqueuse est obtenue à partir de l'eau de mer par les techniques connues sous le terme dessalage et, éventuellement, enrichie en au moins un de ses ions.

4. Utilisation selon la revendication 1 dans laquelle la solution ionique aqueuse est obtenue artificiellement à partir de sels marins.

5. Solution ionique aqueuse obtenue à partir d'eau de mer **caractérisé par** :

   ■ une valeur de pH de préférence inférieure ou au plus égale aux valeurs les plus basses du pH de l'eau de mer,
   ■ une osmolalité inférieure à celle de l'eau de mer et
   ■ une composition du point de vue ionique qui est qualitativement et quantitativement celle de l'eau de mer, à l'exception du point de vue quantitatif, d'une part, de la concentration en potassium qui est supérieure à celle de l'eau de mer et, d'autre part, des concentrations en Na, Mg, Ca et Cl qui sont inférieures à celles de l'eau de mer, lesdites concentrations étant

   - pour $Na^+$, de 1300 à 1500, de préférence de 500 à 1000 mg/l,
   - pour $K^+$, de 4500 à 6500, de préférence de 5000 à 6000 mg/l,
   - pour $Mg^{++}$, de 50 à 1300, de préférence de 100 à 500 mg/l,
   - pour $Ca^{++}$, de 20 à 350, de préférence de 40 à 200 mg/l,
   - pour Cl-, de 4000 à 6000, de préférence de 4500 à 5000 mg/l.

7

**6.** Produits ophtalmiques pour l'hygiène de l'oeil et de ses annexes à base d'une solution ionique aqueuse suivant la revendication 5.

**7.** Utilisation selon la revendication 1 de la solution ionique aqueuse selon la revendication 5.

**8.** Utilisation des solutions ioniques aqueuses obtenues à partir de l'eau de mer, dont la composition ionique est du point de vue qualitatif celle de l'eau de mer et du point de vue quantitatif telle que d'une part leur pH soit de 4 à 9, de préférence de 7 à 8 et que d'autre part leur osmolalité soit de 150 à 700, de préférence de 250 à 350 mOsm/kg pour la préparation d'un médicament destiné au traitement de l'oeil et de ses annexes.

**9.** Utilisation de la solution ionique aqueuse selon la revendication 5 pour la préparation d'un médicament destiné au traitement de l'oeil et de ses annexes.


**Claims**

**1.** Non-therapeutical use for cleaning the eye and its appendages, of aqueous ionic solutions obtained from seawater whose ionic composition is, from the qualitative point of view, that of seawater, and from the quantitative point of view such that, on the one hand, their pH is from 4 to 9, preferably from 7 to 8, and, on the other hand, their osmolality is from 150 to 700, preferably from 250 to 350 mOsm/kg.

**2.** The use as claimed in claim 1, in which the aqueous ionic solution is obtained by diluting seawater, in particular with distilled water, especially at the rate of 2 to 5 fold.

**3.** The use as claimed in claim 1, in which the aqueous ionic solution is obtained from seawater by techniques known by the term desalination and, optionally, enriched with at least one of its ions.

**4.** The use as claimed in claim 1, in which the aqueous ionic solution is artificially obtained from sea salts.

**5.** An aqueous ionic solution obtained from seawater, **characterized by**: a pH value preferably less than or at most equal to the lowest pH values of seawater, an osmolality lower than that of seawater, and a composition, from the ionic point of view, which is qualitatively and quantitatively that of seawater, with the exception from the quantitative point of view, on the one hand, of the potassium concentration which is greater than that of seawater and, on the other hand, of the Na, Mg, Ca and Cl concentrations which are less than those of seawater, said concentrations being

- for $Na^+$ , from 1300 to 1500, preferably from 500 to 1000 mg/l,
- for $K^+$ , from 4500 to 6500, preferably from 5000 to 6000 mg/l,
- for $Mg^{++}$ , from 50 to 1300, preferably from 100 to 500 mg/l,
- for $Ca^{++}$ , from 20 to 350, preferably from 40 to 200 mg/l,
- for $Cl^-$ , from 4000 to 6000, preferably from 4500 to 5000 mg/l.

**6.** Ophthalmic products for cleaning the eye and its appendages based on an aqueous ionic solution as claimed in claim 5.

**7.** The use according to claim 1 of the aqueous ionic solution according to claim 5.

**8.** The use of aqueous ionic solutions obtained from seawater, whose ionic composition is, from the qualitative point of view, that of seawater, and from the quantitative point of view such that, on the one hand, their pH is from 4 to 9, preferably from 7 to 8, and, on the other hand, their osmolality is from 150 to 700, preferably from 250 to 350 mOsm/kg, for the manufacture of a drug for treating the eye and its appendages.

**9.** The use of the aqueous ionic solution according to claim 5, for the manufacture of a drug for treating the eye and its appendages.


**Patentansprüche**

**1.** Nicht therapeutische Verwendung von aus Meerwasser erhaltenen wässrigen ionischen Lösungen, deren ionische Zusammensetzung in qualitativer Hinsicht diejenige von Meerwasser ist und in quantitativer Hinsicht so beschaffen ist, dass einerseits ihr pH 4 bis 9, vorzugsweise 7 bis 8, beträgt und dass andererseits ihre Osmolalität 150 bis 700, vorzugsweise 250 bis 350 mOsm/kg, beträgt, für die Hygiene des Auges und der Augenanhangsorgane.

2. Verwendung nach Anspruch 1, bei der die wässrige ionische Lösung durch insbesondere 2 bis 5fache Verdünnung von Meerwasser insbesondere mit destilliertem Wasser erhalten wird.

3. Verwendung nach Ansnpruch 1, bei der die wässrige ionische Lösung mit Hilfe der unter dem Begriff Entsalzung bekannten Technik aus Meerwasser erhalten und ggf. hinsichtlich mindestens einem ihrer Ionen angereichert wird.

4. Verwendung nach Anspruch 1, bei der die wässrige ionische Lösung künstlich aus Meersalzen erhalten wird.

5. Aus Meerwasser erhaltene wässrige ionische Lösung, **gekennzeichnet durch**:

einen pH-Wert vorzugsweise kleiner als oder höchstens
gleich den niedrigsten Werten des pH-Wert von Meerwasser,
eine Osmolalität, die niedriger als die von Meerwasser ist, und
eine Zusammensetzung in ionischer Hinsicht, die qualitativ und quantitativ diejenige von Meerwasser ist, mit Ausnahme in quantitativer Hinsicht einerseits der Kaliumkonzentration, die höher als diejenige von Meerwasser ist, und andererseits der Konzentrationen an Na, Mg, Ca und Cl, die niedriger als diejenigen des Meerwassers sind, wobei diese Konzentrationen betragen:

- bei $Na^+$ 1300 bis 1500, vorzugsweise 500 bis 1000 mg/1,
- bei $K^+$ 4500 bis 6500, vorzugsweise 5000 bis 6000 mg/1,
- bei $Mg^{++}$ 50 bis 1300, vorzugsweise 100 bis 500 mg/l
- bei $Ca^{++}$ 20 bis 350, vorzugsweise 40 bis 200 mg/l
- bei Cl- 4000 bis 6000, vorzugsweise 4500 bis 5000 mg/l.

6. Augenprodukte für die Hygiene des Auges und der Augenanhangsorgane auf der Basis einer wässrigen ionischen Lösung nach Anspruch 5.

7. Verwendung nach Anspruch 1 der wässrigen ionischen Lösung nach Anspruch 5.

8. Verwendung von aus Meerwasser erhaltenen wässrigen ionischen Lösungen, deren ionische Zusammensetzung in qualitativer Hinsicht diejenige von Meerwasser ist und in quantitativer Hinsicht so beschaffen ist, dass einerseits ihr pH-Wert 4 bis 9, vorzugsweise 7 bis 8, beträgt und dass andererseits ihre Osmolalität 150 bis 700, vorzugsweise 250 bis 350 mOsm/kg beträgt, für die Herstellung eines Medikaments, das für die Behandlung des Auges und der Augenanhangsorgane bestimmt ist.

9. Verwendung von wässriger ionischer Lösung nach Anspruch 5 für die Herstellung eines Medikaments, das für die Behandlung des Auges und der Augenanhangsorgane bestimmt ist.

**EP 1 242 098 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 92300 F

**Littérature non-brevet citée dans la description**

- Handbook of Chemistry and Physics. CRC PRESS, 1982